# EUROPEAN PATENT SPECIFICATION

(11) **EP 3 119 419 B1**
(45) Date of publication and mention of the grant of the patent: **03.04.2019**
(21) Application number: 15713662.3
(22) Date of filing: 17.03.2015
(51) Int. Cl.: A61K 38/20, A61K 38/19, A61K 45/06, A61K 47/10, A61K 8/90, A61K 9/00, A61P 35/00

(54) **COMBINATION FOR USE IN A METHOD OF TREATING CANCER**
KOMBINATION ZUR VERWENDUNG BEI VERFAHREN ZUR BEHANDLUNG VON KREBS
COMBINAISON POUR UNE UTILISATION DANS UN PROCÉDÉ DE TRAITEMENT DU CANCER

(30) Priority: 17.03.2014 EP 14160262
(43) Date of publication of application: 25.01.2017
(73) Proprietor: Jachimczak, Piotr, 97074 Würzburg (DE)
(72) Inventor: AIGNER, Achim, 04105 Leipzig (DE); JACHIMCZAK, Piotr, 97074 Würzburg (DE)
(74) Representative: V.O.
(86) International application number: PCT/EP2015/055528
(87) International publication number: WO 2015/140150

(56) References cited:
- WO-A1-2013/155487
- CLAUDIA PENAFUERTE ET AL: "TGFÎ secreted by B16 melanoma antagonizes cancer gene immunotherapy bystander effect", CANCER IMMUNOLOGY, IMMUNOTHERAPY, SPRINGER, BERLIN, DE, vol. 57, no. 8, 24 January 2008 (2008-01-24), pages 1197-1206, XP019624377, ISSN: 1432-0851
- JASON PARK ET AL: "Combination delivery of TGF-[beta] inhibitor and IL-2 by nanoscale liposomal polymeric gels enhances tumour immunotherapy", NATURE MATERIALS, vol. 11, no. 10, 15 July 2012 (2012-07-15) , pages 895-905, XP055133368, ISSN: 1476-1122, DOI: 10.1038/nmat3355
- WOJTOWICZ-PRAGA S ET AL: "Modulation of B16 melanoma growth and metastasis by anti-transforming growth factor beta antibody and interleukin-2.", JOURNAL OF IMMUNOTHERAPY WITH EMPHASIS ON TUMOR IMMUNOLOGY : OFFICIAL JOURNAL OF THE SOCIETY FOR BIOLOGICAL THERAPY MAY 1996, vol. 19, no. 3, May 1996 (1996-05), pages 169-175, XP009179544, ISSN: 1067-5582
- YANG L ET AL: "TGF-beta and immune cells: an important regulatory axis in the tumor microenvironment and progression", TRENDS IN IMMUNOLOGY, ELSEVIER LTD. * TRENDS JOURNALS, GB, vol. 31, no. 6, 1 June 2010 (2010-06-01), pages 220-227, XP027079217, ISSN: 1471-4906, DOI: 10.1016/J.IT.2010.04.002 [retrieved on 2010-06-01]
- S. H. WRZESINSKI ET AL: "Transforming Growth Factor- and the Immune Response: Implications for Anticancer Therapy", CLINICAL CANCER RESEARCH, vol. 13, no. 18, 15 September 2007 (2007-09-15), pages 5262-5270, XP055133395, ISSN: 1078-0432, DOI: 10.1158/1078-0432.CCR-07-1157
- RICHARD A. FLAVELL ET AL: "The polarization of immune cells in the tumour environment by TGF[beta]", NATURE REVIEWS IMMUNOLOGY, vol. 10, no. 8, 1 August 2010 (2010-08-01) , pages 554-567, XP055133391, ISSN: 1474-1733, DOI: 10.1038/nri2808
- BROOKS STEPHEN ET AL: "Intratumoral injection of GM-CSF in perspective - a review.", JOURNAL OF MEDICINE 2003, vol. 34, no. 1-6, 2003, pages 149-153, XP009185114, ISSN: 0025-7850
- KABANOV ALEXANDER V ET AL: "PLURONIC BLOCK COPOLYMERS IN DRUG DELIVERY: FROM MICELLAR NANOCONTAINERS TO BIOLOGICAL RESPONSE MODIFIERS", CRITICAL REVIEWS IN THERAPEUTIC DRUG CARRIER SYSTEMS, BEGELL HOUSE PUBLISHING INC, US, vol. 19, no. 1, 1 January 2002 (2002-01-01), pages 1-72, XP009082062, ISSN: 0743-4863, DOI: 10.1615/CRITREVTHERDRUGCARRIERSYST.V19.I1. 10

## Description

The present invention refers to a combination of two immune-stimulating cytokines which are IL-2 and/or GM-CSF, combined with a TGF-beta inhibitor for use in a method of treating cancer comprising real-time, intratumoral, presentation of personalized tumor-associated antigens (pTAAs) in growing tumor tissue of cancer-bearing patients (4-D Vaccine). In a further embodiment, the invention refers to a pharmaceutical composition comprising this combination and a pharmaceutically acceptable carrier.

### Background

Cancer is one of the major causes for death in the world. Novel immune therapeutic strategies show first promising results in patients. However, the high rate of non-responding tumor cells or the cancer recurrance after treatment still remains a serious problem for the development of new effective therapies. The reason for disappointing clinical results is closely related to the **Intratumoral Heterogeneity,** which describe extremely accelerated phenotypic differentiation of rapidly cycling cells, due to Darwinian selection process leading to dynamic heterogeneity of Tumor Associated Antigens (TAAs) expressed by proliferating tumor cells and to the **Intratumoral Immunosuppression.** Although, through evolutionary development, the human immune system is capable of successfully fighting off a large number of diseases including malignant cancers, nevertheless, it is not in all cases able to efficiently combat the abnormal cells. The most important reason for this is the so-called "immune-escape phenomenon", which involves mechanisms that help the tumor to deceive the immune system and evade immune reactions. The key factor driving "immune-escape phenomenon" is the release of the immunosuppressive messenger protein Transforming Growth Factor-beta (TGF-beta). Overexpressed and locally activated TGF beta in cancer suppresses the ability of the immune system to recognize the TAAs, indeed it also inhibits the development of an effective anti-tumor immune response leading to an undisturbed (re-) growth and clinical progression of the tumor.

### Tumor Heterogenity

Most of the current experimental models of cancer do not reflect the *"in vivo"* complexity of naturally occurring tumors. The vast majority of human cancers arises sporadically and is clonal in origin, and progresses through sequential accumulation of somatic mutations and/or epigenetic changes in genes, whose gain or loss of function is associated with malignant transformation.

There are two major models of cancer development and progression to metastatic disease (Marjanovic et al., 2012). The clonal evolution based on Darvinian selection theory suggests that successive mutations accumulating in a given cell generate clonal outgrowths that thrive in response to microenvironmental selection pressure, dictating the continuously changing phenotype of the growing tumor. The alternative cancer stem cell (CSC) model postulate that cancer cells with similar genetic backgrounds can be hierarchically organized according to their tumorigenic potential. Evolving evidence supports a new model of tumorigenicity, in which considerable plasticity between both non-CSC and CSC models exist and is further affected by interactions with numerous non-malignant cell types recruited in a growing tumor stromal tissue from new blood vessels (Bindea et al., 2013).

Multiregion genetic analysis of tumor biopsies provided evidence of intratumoral heterogeneity in all tumors, with spacially separated heterogeneous somatic mutations and chromosomal imbalances leading to phenotypic intratumoral diversity amongst the cancer cells themselves (Gerlinger et al., 2012). Genetic mutations underlying primary tumor formation are a major source of cell-intrinsic variability with a profound impact on cell phenotype. However, the genetic mutations are not the sole determinants of cancer heterogeneity. Multistep tumorigenesis disrupts a natural epigenetic differentiation program of a normal cell of origin, which additionally influences the phenotype of a growing malignant cell. Therefore, the cell-intrinsic features must be considered in a context-dependent manner. Cell behavior is strongly influenced by factors in the microenvironment acting to promote or inhibit the aggressive cancer phenotype.

Whilst cancer cells cannot anticipate future evolutionary events, the immune system must be managed to recognize all phenotyping changes in real-time mode and guide tumor destruction by inducing specific, host anti-tumor immune responses. In this context, understanding the key processes involved in the interplay between a rapidly growing tumor and the host's immune system is fundamental for optimizing current treatment strategies.

### Cancer immunoediting

In a simple immune response model of cancer, increased tumor antigenicity should always lead to enhancement of immune responses against tumor cells. In the course of carcinogenesis, however, when a tumor reaches a clinical size in the order of 10⁹-10¹¹ cells, there are several mechanisms by which the tumor may have escaped the immunosurveillance (Jamal-Hanjani et al., 2013). One possibility is that the antigens presented by the tumor are not strong or prevalent enough to elicit an immune response. In this case, the tumor is not recognized as a significant threat by the immune system. Another possibility is that the antigen may be present in sufficient strength, but the immunosuppressive elements may diminish the efficacy of the immune system (Prehn et al., 1989). According to a mathematical model presented by Robertson-Teesi et al., there is an optimal antigenicity for all fast-growing tumors. If they are minimally antigenic, then there is only a small immune response; if they are highly antigenic, there may be not only a large immune response, but also a large suppressive response by overexpression of TGF-beta. In between these two scenarios, the immune response may be large enough to affect the tumor, but avoid excessive promotion of Tregs and other suppressive elements like TGF-beta (Robertson-Tessi et al., 2012).

The cancer immunoediting hypothesis, as described in short above, explains why despite years of preclinical efforts and hundreds of immunologic-based clinical studies, the ability of immunotherapies to reduce tumor growth in humans are still limited (Schreiber et al., 2011). Current therapeutic manipulations of immune checkpoints like blockade of CTLA-4 or PD-1/PD-L1, provide first success in some therapeutic settings; however, due to the complexity of the interactions between developing tumors and the immune system, the majority of cancer immunotherapies still need further improvement as proposed here by use of dynamic "Real-Time Tumor Vaccination".

### TGF-beta as a key driver of "Immune-Escape" in advanced, late-stage cancer

### Antagonization of the TGF-beta system in cancer

Human TGF-beta protein family consists preferably of three isotypes: TGF-betal, TGF-beta2, and TGF-beta3 being differentially overexpressed in malignant tumors. Many recent studies favor the hypothesis that blocking TGF-beta-induced signaling in the tumor microenvironment enhances antitumor immunity and may be beneficial for cancer therapy (Jachimczak et al., 1993, Flavell et al., 2010). During the past two decades some important studies have highlighted the therapeutic potential of antagonizing the TGF-beta pathway in cancer (Akhurst et al., 2012). Many drugs that target TGF-beta function have been developed so far. Some of them have reached Phase III clinical trials for a number of disease indications including cancer (see Table 1). However, due to the complex biology of TGF-beta which is highly context-dependent and influenced by the cell type, disease stage *in vivo* and innate genetic variation among individuals, the development of the best strategy to target this system in humans is still very challenging (Connolly et al., 2012).

The major classes of TGF-beta inhibitors include ligand traps, antisense oligonucleotides (ASO), small molecule receptor kinase inhibitors and peptide aptamers (see e.g., Table 1). Ligand traps include anti-ligand neutralizing antibodies and soluble decoy receptor proteins that incorporate the ectodomains from either TGF-beta receptor II (TβRII) or TGF-beta receptor III (TβRIII)/betaglycan protein. Neutralizing antibodies have been raised against individual ligands or may be designed to block all three human isomers TGF-beta 1, -2 and -3. ASO can also be used to reduce the bioavailability of active TGF-beta ligands by blocking *de novo* synthesis of TGF-beta. ASOs are single-stranded polynucleotide molecules, usually 13-25 nucleotides in length, that hybridize to complementary RNA, inhibiting mRNA function, and preventing protein synthesis through accelerated mRNA degradation by RNase H or steric blockade.

Another therapeutic strategy is to block TGF-beta receptor I (TβRRI) activity through the use of small molecule receptor kinase inhibitors that act via ATP-competitive inhibition of the catalytic domain of the serine-threonine kinase of the TβRI (ALK5-Receptor). Lastly, targeting intracellular TGF-beta signaling molecules, such as Smads, is possible with the use of peptide aptamers. Aptamers are small peptide, DNA or RNA molecules containing a target-binding and a scaffolding domain that stabilize and interfere with the function of the target.

All anti-TGF-beta strategies described above suffer from several limitations with respect to their delivery, specificity and/or toxicity. However, their most crucial and common limitation by far is the fact that TGF-beta signaling is involved in many different normal physiological functions, and that normal tissues are as equally compromised as tumors upon systemic effects of anti-TGF-beta therapies. Therefore, the systemic neutralization of this otherwise so important growth factor is associated with the risk of severe side-effects. To overcome this issue, we invented local (i.e. intratumoral) application of the TGF-beta inhibitor in order to achieve efficient concentrations of the anti-TGF-beta drug in the growing tumor tissue, while the resulting systemic concentrations are far too low to cause any dose-limiting or clinically relevant toxicological effect (Hau & Jachimczak et al., 2007, Bogdahn et al., 2012).

Penafuerte et al. (Cancer Immunol Immunother (2008) 57: 1197-1206) investigate the bystander effect of a GMCSF/IL-2 fusion protein in B16 cells which is revived and improved, respsectively, by co-administration of a TGF-beta inhibitor.

Park et al. (Nature Materials, Vol. 11, Oct. 2012) describe the combination of a TGF-beta inhibitor and IL2 via nanoscale liposomal polymeric gels to enhance tumor therapy.

### Real-time approach to generate a tumor vaccine in vivo ("Online Vaccine")

The key element of the present invention involves *"de novo"* generation of effective anti-tumor immune response in cancer-bearing patients, characterized by a high antigenic specificity due to a continued scanning of the patient's personal tumor-associated antigens (pTAAs) profile in a real-time ("online scan") in a TGF-beta-free environment. Such dynamic vaccination approach or in other words four-dimensional (4D) vaccine may be used to treat particularly advanced, late-stage, cancer by use of repetitive spacio (intratumoral) - temporal (real-time) therapy comprising or consisting of a TGF-beta Inhibitor with a particular combination of immunostimulating cytokines such as IL-2 and/or GM-CSF. Due to a local, intratumoral drug application the present invention overcomes all disadvantages of former combinations or pharmaceutical compositions for use in treating cancer and thus, provides a key impact for improved immunotherapy of advanced late-stage cancer. Although the side-effects associated with TGF-beta Inhibition are limited to a local region in close proximity to the tumor, the highly specific, TAA-driven anti-tumor immune response is amplified and systemically distributed across the body by the circulating T-cells. Thus, the advantages of the present invention, which employs an effective real-time approach to screen/scan intratumoral, all individually-specific TAA in a TGF-beta-free milieu, are that 1) the invention overcomes the disadvantages of former combinations or pharmaceutical compositions used to treat cancer and 2) the invention has a key impact on improved immunotherapy of advanced malignant cancer.

### Summary

The present invention relates to a unique, spacio-temporal application of a combination of an immune-stimulating cytokine, e.g. at least two of immune-stimulating cytokines such as IL-2 and GM-CSF with a TGF-beta inhibitor such as an ALK5 inhibitor for use in a method of treating advanced, late-stage human cancer, wherein the TGF-beta inhibitor and IL-2 and GM-CSF are injected intratumorally. In a first aspect the method involves **INTRATUMORAL** reduction of TGF-beta activity by blockade of TGF-beta signaling for example through the type I TGF-beta receptor kinase ALK5 (e.g., SD 208), wherein a local inhibition of a TGF-beta function in a growing tumor guides the immune system to scan all currently expressed personalized pTAAs in real-time by antigen-presenting cells (APC) from intratumoral lymphoid structures. In a second aspect, tumor tissue being exposed to sufficient TGF-beta inhibition activates intratumorally tertiary lymphoid structures to generate effective anti-tumor immune response. This includes both: induction of APC function by e.g. GM-CSF-treated CD103+ Dendritic Cells (DCs) as well as cytotoxic CD8+ T Lymphocytes by Interleukin 2-treated T cells in a TGF-beta-free melieu.
The simultaneous **INTRATUMORAL** application of both therapeutic regimens including TGF-beta Inhibition with immunostimulation e.g., by combined use of IL-2 and GM-CSF polarized the development of TGF-driven suppressive and/or toloregenic immune phenotype consisting of immunosuppressive T regulatory cells (Treg) and monocyte-derived suppressor cells (MDSC) results into a specific anti-tumor cytotoxic immune response.
Figures 1A to 1C present the evaluation of growth-modulatory effects of SD208, IL-2 and GM-CSF upon PBMC proliferation, as measured with trypan blue exclusion assay.
Figure 2 shows the evaluation of effects of components of the present invention SD-208+IL-2+GM-CSF upon allogenous cell cytotoxicity against human glioma cells HTZ-349 and A-172 in a calcein-release-cell-cytotoxicity assay (CARE-LASS).
Figure 3 presents an *in vivo* B16-mouse model of intratumoral delivery of SD208 in a poloxamer complex with DMSO.
Figures 4A to 4C depict different models of intratumoral injection, wherein Fig. 4A refers to one intratumoral injection, Fig. 4B to two separate intratumoral injections and Fig. 4C to two separate intratumoral/intraparenchymal injections.

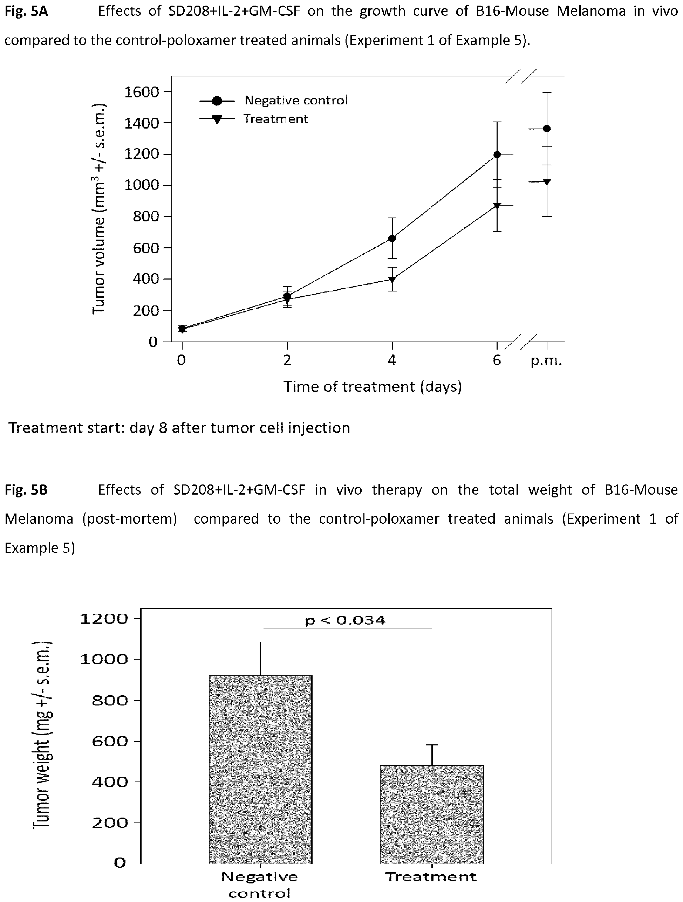

Figures 5A and 5B present an *in vivo* B16-mouse model treated with pharmaceutical components of RealTVac®, i.e., a combination of an ALK5 inhibitor such as SD208, IL-2 and GM-CSF. Fig. 5A presents the effects of the combination of SD208, IL-2 and GM-CSF on the tumor volume in the B16-mouse melanoma in comparison to a negative control treated with poloxamer which results in a reduction of the tumor volume of about 35 %. Fig. 5B shows the effect of a combination of SD208, IL-2 and GM-CS on the total weight of B16-mouse melanoma compared to poloxamer treated control animals which results in about 45 % reduction of the tumor weight.
Figure 6 shows the effects of a combination of SD208 and IL-2 in comparison to the effects of a combination of SD208, IL-2 and GM-CSF, wherein the combination of three compounds presents a stronger effect (about 50 %).

### Detailed description

Essential elements of the present invention, which is a four-dimensional (4-D) approach for effective presentation of personal tumor associated antigens (pTAA) *in vivo,* is a combined administration in a spacial (intratumoral) and temporal (real-time) context of a TGF-beta inhibitor and IL-2 and GMCSF. The four-dimensional (4-D) approach is defined as unification of space (three-dimensional tumor) and time (presentation of currently expressed pTAA profile in real-time mode, wherein the profile represents the pheonotype of the tumor).

### 1) Inhibitor of TGF-beta:

A TGF-beta inhibitor of the present invention interacts with a TGF-beta receptor, inhibits the signaling of the receptor, interacts with a TGF-beta protein, and/or inhibits the transcription and/or translation of the gene encoding TGF-beta-1, -2, and/or -3. The TGF-beta inhibitor of the present invention inhibits TGF-betal, TGF-beta2, and/or TGF-beta3; in case an inhibitor inhibits all three TGF-beta isotypes, the inhibitor is a pan-specific inhibitor. Examples of TGF-beta inhibitors of the present invention are:
A) a small molecule inhibitor of TGF-beta receptor type I kinase (ALK5; an inhibitor is an ALK5 inhibitor),
B) a neutralizing anti-TGF-beta-1, -2, -3 antibody or TGF-beta binding fragments thereof,
C) a neutralizing anti-TGF-beta receptor type I,- type II or type III antibody or TGF-beta receptors binding fragments thereof,
D) an antisense oligonucleotide specific for mRNA encoding TGF-betal, -2, and -3 isotypes or other components of TGF-beta signaling assembly optionally comprising a modified nucleoside such as 2'-O, 4'-C-methylene linked bicyclic ribonucleotides, known as locked nucleic acids LNA (e.g., oxy-LNA, amino-LNA, thio-LNA), phosphorodiamidate morpholino oligomers (PMO), phosphorothioate (PS), 2"-O-methyl (2'-Ome), 2'-fluoro (2'-fluoro (2'-F), or 2'-methoxyethyl (2'-MOE) derivatives,
E) an antisense RNA molecule specific for TGF-beta2-mRNA like belagenpumatucel-L and/or TGF-beta1-mRNA or TGF-beta3-mRNA or other components of mRNA encoding TGF-beta signaling assembly,
F) a silencing RNA molecule (siRNA) specific for mRNA encoding TGF-betal, -2, and/or -3 isotypes or other components of TGF-beta signaling assembly,
G) a short hairpin RNA (shRNA) specific for mRNA encoding TGF-betal, -2, and/or -3 isotypes or other components of TGF-beta signaling assembly,
H) a miRNA molecule specific for mRNA encoding TGF-betal, -2, and/or -3 isotypes or other components of TGF-beta signaling assembly,
I) an aptamer and/or spiegelmer molecule specific for TGF-betal, -2, and/or -3 isotypes or other components of the TGF-beta signanling assembly, and/or
J) a ribozyme molecule specific for mRNA encoding TGF-betal, -2, and/or -3 isotypes or other components of the TGF-beta signanling assembly.

ASOs are single-stranded polynucleotide molecules comprising 13-25 nucleotides, preferably 15-20 nucleotides, more preferred 15, 16, 17, 18, 19, 20, 21, 22 or 23 nucleotides, that hybridize to complementary RNA, inhibiting mRNA function, and preventing protein synthesis for example through accelerated mRNA degradation by RNase H or steric blockade.

In detail, a TGF-beta inhibitor binding to TGF-beta receptor type I, preferably a non-peptide, small molecule inhibitor of ALK-5 kinase from dihydropyrolopyrazole derivatives (e.g., LY2157299, LY21109761, LY580276, LY550410, GW788388), pyrazole derivatives (e.g., LY364947, HTS-466284, SM305, SB525334, A-83-01), quinazoline derivatives (e.g., SD-208), or imidazole derivatives (e.g., SM16, SB431542, or SB505124). SD-208 for example is a small molecule inhibitor of type I kinase of TGF-beta receptor with IC₅₀ = 49 nM, which inhibits TGF-beta signal transduction in a dose-dependent fashion, as for example measured by p-SMAD2 western blot analysis.

A human pan-anti-TGF-beta antibody binding to and neutralizing all three isotypes of TGF-beta (e.g., TGF-betal, 2, 3) like GC-1008, a neutralizing high-affinity antibody or antigen-binding immunoglobulin single variable domain or polypeptide thereof, that neutralize mature human TGF-beta1 (like CAT 192), or hTGF-beta2 (like CAT 152), and neutralizing anti-TGF-beta receptor type II antibody (like D10) or an antibody fragment thereof which binds and neutralizes human TGF-beta receptor type II, a fusion protein comprising a TGF-beta type II receptor fusion protein, a small molecule or peptide consisting of, for example, 11-50 amino acid residues blocking assembly of the TGF-beta signaling complex extra- and intracellularly.

An amino acid-blocking assembly is a small polypeptide physically interacting with target protein(s) by electrostatic forces, leading to its (their) functional failure.

The present invention comprises administration of an effective amount of an iRNA agent, including sense and antisense sequences capable of forming an RNA duplex. The sense sequence of the iRNA agent preferably includes a nucleotide sequence substantially identical to a target sequence of about 19 to 23 nucleotides of TGF-beta-mRNA, and the antisense sequence preferably includes a nucleotide sequence complementary to a target sequence of about 19 to 23 nucleotides of TGF-beta mRNA. Further TGF-beta inhibitory drugs are summarized in **Table 1:**

### Table 1

**Table 1: Summary of TGF-beta inhibitory drugs in clinical and preclinical development according to Akhurst & Hata, 2012.**

| ***Drug*/*Company*** | ***Type*/*Targets*** | ***Stage*** | ***Clinical indication*** |
|---|---|---|---|
| | | | |
| Trabedersen, Antisense Pharma | PS-ODNs TGF- β2 | Phase III | Malignant Cancer |
| Belagenpumatucel-L, NovaRx | AS-mRNA TGF-β2 | Phase III | NSCLC |
| Disitertide(P144), Digna Biotech | Peptide (TGFRIII fragment) | Phase II | Systemic Sclerosis |
| Lerdelimumab(CAT-152), Genzyme | Antibody TGF-β2 | Phase III | Glaucoma |
| Metelimumab (CAT-192), Genzyme | Antibidy TGF-β1 | Phase II | Systemic Sclerosis |
| Fresolumimab(GC-1008), Sanofi | Antibody TGF-β1/2/3 DNA | Phase I | Sclerosis, Cancer |
| FANG™ Vaccine with bi-siRNAfurin | DNA Plasmid | Phase III | Malignant Cancer |
| LY2382770, Eli Lilly | Antibody TGF-β1 | Phase II | Kidney Fibrosis |
| SIX-1ββ, Stromedix | Antibody aVβ6 intergrin | Phase II | Fibrosis |
| Ly2157299, Eli Lilly | small molecule ALK5 TβRII | Phase II | Malignant Cancer |
| Dominant neg. TGFβR2-mod.T cells | Recombinant T cells | Phase I | Lung Cancer, Lymphoma, |
| Avotermin(Juvista), Renova | Recomb. Protein TGFβ3 | Phase II | Scarring |
| Pirfenidone, InterMune | Small Molecule TGF-β | Phase III | Fibrosis, Scarring |
| Losartan(AT1), Merck | Small Molecule TGF-β | Phase I/II | Marfan Syndrom |
| Tranilast, Kissei Pharmaceuticals | Small Molecule TGF-β | Phase III | Corneal Pterygium |
| IMC-TR1, ImClone/Eli Lilly | Antibody TGF-βRTII | Phase II | Malignant Cancer |
| AP11014, Antisense Pharma | PS-ODNs TGF-1 | Preclinical | Malignant Cancer |
| P17, Digna Biotech | Pept. binding TGF-βR/TGF | Preclinical | Fibrosis, Cancer |
| LSKL, Academic | Peptide Thrombospondin | Preclinical | Diabetic Nephropathy |
| 1D11, R&D Systems | Mouse pan-TGF-β-Antibody | Preclinical | Breast Cancer |
| SR2F, Academic | Ligand Trap TGF-β1+β3 | Preclinical | Breast Cancer |
| soluble TβR2-Fc, Genzyme | Ligand Trap TGF-β1+β3 | Preclinical | Breast Cancer |
| LY580276, LY550410, Eli Lilly | Small Molecule ALK5 TβRI | Preclinical | Cancer |
| SD-505124, SB-431542, GSK | Small Molecule ALK5 TβRI | Preclinical | Cancer |
| SD208, SD093, Scios | Small Molecule ALK5 TβRI | Preclinical | Cancer |
| Ki26894, Lirin Pharmaceuticals | Small Molecule ALK5 TβRI | Preclinical | Breast Cancer |
| SM16, Biogen Idec | Small Molecule ALK5 TβRI | Preclinical | Cancer |
| GW788388, GlaxoSmithKline | Small Molecule ALK5 TβRI | Preclinical | Fibrosis |
| GB1201, GB1203, Academic | Pyrrole-imidazole TGFβ1 | Preclinical | Fibrosis |
| ASPH_1047/0047, Isarna | LNA-ODNs TGF-β1/TGF-β2 | Preclinical | Cancer, Fibrosis |
| ALK5-Inhibitor, Rigel/BMS | Small molecule ALK5 TβRI | Preclinical | Cancer |

### 2) Immune-stimulating cytokines: Interleukin-2 (IL-2) and/or Granulocyte-Macrophage Colony Stimulating Factor (GM-CSF)

IL-2 (Interleukin-2, aldesleukin) is a T helper1 (Th1) cytokine that plays a key role in the activation and proliferation of lymphocytes that have been primed by antigens (e.g, TAA). IL-2 is pivotal for the expansion of most T cells, NK cells, and B cells during various phases of their antigen response. IL-2 is secreted during T cell activation and fuels the growth of activated T cells. Apart from its most important role to mediate antigen-specific T lymphocyte proliferation, IL-2 also modulates the expression of IFNγ and MHC antigens, stimulates proliferation and differentiation of activated B cells, and augments NK cell activity. IL-2 signals through various signaling pathways that include the activation of JAK/STAT5, RAS/MAPK and the PI3 kinase pathways. These activations make IL-2 a known potent T cell growth factor, inducer of lymphokine-activated killer activity, boosts the cytolytic activity of NK cells, augmentation of immune globulin (Ig)gt production, and an essential factor for the development of Treg cells (e.g., when used with TGB-beta).

GM-CSF (granulocyte-macrophage colony stimulating factor, such as sargramostin/molgramostin)(Leukine/USA-Leucomax/EU), is a pleiotropic, glycosylated growth factor produced by a wide variety of tissue types, including fibroblasts, endothelial cells, T cells, macrophages, mesothelial cells epithelial cells and various tumor types. The biological effects of GM-CSF are mediated through its binding to cell surface receptors that are widely expressed on hematopoietic cells, as well as some non-hematopoietic cells such as endothelial cells. GM-CSF stimulates proliferation, activation, and differentiation of myeloid dendritic cells (mDCs), intratumoral CD103+ dendritic cells, macrophages, granulocytes, neutrophils and eosinophils. GM-CSF can also enhance several T-cell stimulatory activities of DCs, including antigen uptake, major histocompatibility complex (MHC) activity, costimulatory molecule expression and interleukin 12 (IL-12) production. It may also enhance tumor invasion by upregulation of MMP-2 and -9 and -26 in cancer. Moroever, in TGF-beta environment, GM-CSF drives generation of MDSC. In most tissues, inflammatory mediators, such as IL-1, IL-6, TNFα, or endotoxin, are inducers of GM-CSF gene expression. GM-CSF was shown to be the most effective molecule in inducing antitumor reactivity (Dranoff 2003; Broz et al., 2014).

The present invention refers for example to a combination of IL-2 and/or GM-CSF with an ALK-5 inhibitor such as SD-208 for use in a method for the treatment of cancer, particularly in the local, e.g., intratumoral therapy of advanced, grade 3 or 4, late-stage and/or metastatic cancers such as melanoma, gastric carcinoma, colon carcinoma, rectal carcinoma, hepatocellular carcinoma, cholangiocarcinoma, pancreatic carcinoma, esophageal carcinoma, other adenocarcinoma, breast cancer, prostatic cancer, kidney cancer, non-small cell lung cancer, small cell lung cancer, retinoblastoma, ovarian carcinoma, cervix carcinoma, osteosarcoma, chondrosarcoma, leiomyosarcoma, rhabdomyosarcoma, mesothelioma, fibrosarcoma, agiosarcoma, liposarcoma, glioma, myxosarcoma, mixed connective tissue tumor, non-Hodgkin or Hodgkin lymphoma. Additionally, all combinations of the present invention are also for use in a method of treating metastasis of the above-mentioned malignant (primary) tumors.

The immune-stimulating cytokine such as IL-2 and/or GM-CSF and the ALK-5 inhibitor such as SD-208 are injected separately or combined in one product, for example, in a pharmaceutical composition, optionally comprising a pharmaceutically acceptable carrier. If the compounds are injected separately, they are injected consecutively, preferably in a defined time period, which can be e.g, 1 min. or 5 min. to 12 h, 30 min. to 6 h, or 1 to 2 h. In another alternative, the compounds of the combination, e.g., IL-2 and GM-CSF and the ALK-5 inhibitor such as SD-208, are combined right before the intratumoral injection, for example in one injection. In an embodiment, all compounds of the combination or pharmaceutical composition show their treating effects at the same time.

The intratumoral concentration of the immune stimulator IL-2 is for example in a range of 1.8x10⁶ to 18x10⁶ IU, and/or the intratumoral concentration of the immune stimulator GM-CSF is in a range of 0.1-0.5mg; in such combination, the intratumoral concentration of the ALK5 inhibitor is for example in a range of about 1 nM to about 10 mM, or 0,1 µM to 1 mM.

The immune stimulatorslL-2 and GM-CSF, and the TGF-beta inhibitor, e.g. ALK-5 inhibitor are optionally combined with another TGF-beta-specific DNA- and RNA-targeting compound such as siRNA, shRNA, ribozymes, miRNA, antisense RNA, aptamer, antisense oligonucleotides, spiegelmer etc. All these compounds are administered subsequently, wherein one or more of the compound(s) is administered every day, every week, every month, every three months, every six months, and one or more other compounds are administered every day, every week, every month, every three months, every six months, i.e., in a time lag to the administration of the first, second or third etc. compound. Alternatively, all compounds are administered at the same time, e.g., in the form of a pharmaceutical composition comprising or consisting of the compounds, and optionally comprising a pharmaceutically acceptable carrier. Just like for each compound, the pharmaceutical composition is preferably injected intratumorally.

In another embodiment of the present invention, an additional immunomodulating factor is combined with IL-2 and/or GMCSF and a TGF-beta inhibitor. The additional immunomodulating factor is selected from the following group consisting of:
A) a further immune-stimulating cytokine: IFNα, IFNβ, IFNγ, MIPα, MIPβ, TNFα, G-CSF, M-CSF, MIP, and interleukins (IL) like IL-1α, IL-1β, IL-3, IL-5, IL-6, IL-7, IL-12, IL-15, IL-16, IL-18, IL-21, IL-24, IL-32, IL-33, IL-36 which likewise regulate the function of the immune system,
B) a tumor associated antigen (TAA) such as unique antigens resulting from mutations as presented for example in Table 2, tumor-specific antigens such as presented for example in Table 3, differentiation antigens such as presented for example in Table 4, or overexpressed antigens such as those presented for example in Table 5.
C) an immune-stimulating ligand binding to and activating costimulatory receptors expressed on immune cells, for example, CD40, CD134 (OX40), CD137, orTIM-3,
D) an inhibitor of prostaglandin E (PGE), interleukin 10 (IL-10), and interleukin 35 (IL-35),
E) an inhibitor of immunoglobulin-like transcript (ILT) such as e.g, ILT2 and ILT3,
F) an inhibitor of immunomodulating cytotoxic T-lymphocyte antigen 4 (CTLA-4), e. g. ipilimumab,
G) an inhibitor of glucocorticoid-induced tumor necrosis factor family receptor (GITR),
H) an inhibitor of programmed cell death protein 1 (PD-1), such as e.g, nivolumab or pembrolizumab and respective ligands, i.e. other molecules binding the protein e.g. PD-L1 and/or PD-L2, and/or
I) an inhibitor of melanoma inhibiting activity (MIA).

| ***Table 2*** | ***Tumor Antigens Resulting from Mutations*** |
|---|---|
| date of download: 20140309 | http://cancerimmunity.org/peptide/mutations/ |
| | |

| ***Gene*/*Protein*** | ***Peptide*** |
|---|---|
| | |
| alpha-actinin-4 | FIASNGVKLV |
| ARTC1 | YSVYFNLPADTIYTNh |
| BCR-ABL fusion protein (b3a2) | SSKALQRPV, GFKQSSKAL, ATGFKQSSKALQRPVAS |
| B-RAF | EDLTVKIGDFGLATEKSRWSGSHQFEQLS |
| CASP-5 | FLIIWQNTM |
| CASP-8 | FPSDSWCYF |
| beta-catenin | SYLDSGIHF |
| Cdc27 | FSWAMDLDPKGA |
| CDK4 | ACDPHSGHFV |
| CDKN2A | AVCPWTWLR |
| CLPP | ILDKVLVHL |
| COA-1 | TLYQDDTLTLQAAG |
| dek-can fusion protein | TMKQICKKEIRRLHQY |
| EFTUD2 | KILDAVVAQK |
| Elongation factor 2 | ETVSEQSNV |
| ETV6-AML1 fusion protein | RIAECILGMi, IGRIAECILGMNPSR |
| FLT3-ITD | YVDFREYEYY |
| FN1 | MIFEKHGFRRTTPP |
| GPNMB | TLDWLLQTPK |
| LDLR-fucosyltransferaseAS fusion protein | WRRAPAPGA, PVTWRRAPA |
| hsp70-2 | SLFEGIDIYT, AEPINIQTW |
| MART2 | FLEGNEVGKTY |
| ME1 | FLDEFMEGV |
| MUM-1f | EEKLIVVLF |
| MUM-2 | SELFRSGLDSY |
| MUM-3 | EAFIQPITR |
| neo-PAP | RVIKNSIRLTL |
| Myosin class I | KINKNPKYK |
| NFYC | QQITKTEV |
| OGT | SLYKFSPFPL |
| OS-9 | KELEGILLL |
| p53 | VVPCEPPEV |
| pml-RARalpha fusion protein | NSNHVASGAGEAAIETQSSSSEEIV |
| PRDX5 | LLLDDLLVSI |
| PTPRK | PYYFAAELPPRNLPEP |
| K-ras | VVVGAVGVG |
| N-ras | ILDTAGREEY |
| RBAF600 | RPHVPESAF |
| SIRT2 | KIFSEVTLK |
| SNRPD1 | SHETVIIEL |
| SYT-SSX1 or -SSX2 fusion protein | QRPYGYDQIM |
| TGF-betaRII | RLSSCVPVA |
| Triosephosphate isomerase | GELIGILNAAKVPAD |

| ***Table 3*** | ***Shared Tumor-Specific Antigens*** |
|---|---|
| date of download: 20140309 | http://cancerimmunity.org/peptide/tumor-specific/ |
| | |

| *Gene* | ***Peptide*** |
|---|---|
| | |
| BAGE-1 | AARAVFLAL |
| Cyclin-A1 | SLIAAAAFCLA, FLDRFLSCM |
| GAGE-1,2,8 | YRPRPRRY |
| GAGE-3,4,5,6,7 | YYWPRPRRY |
| GnTVf | VLPDVFIRC(V) |
| HERV-K-MEL | MLAVISCAV |
| KK-LC-1 | RQKRILVNL, NYNNFYRFL |
| KM-HN-1 | EYLSLSDKI, EYSKECLKEF, MLMAQEALAFL, |
| LAGE-1 | SLLMWITQC, MLMAQEALAFL, LAAQERRVPR, LELVRRILSR, APRGVRMAV, QGAMLAAQERRVPRAAEVPR, ILSRDAAPLPRPG AADHRQLQLSISSCLQQL, SLLMWITQCFLPVF, CLSRRPWKRSWSAGSCPGMPHL, AGATGGRGPRGAGAAGATGGRGPRGAGA |
| MAGE-A1 | EADPTGHSY, KVLEYVIKV, SLFRAVITK, EVYDGREHSA, RVRFFFPSL, EADPTGHSY, REPVTKAEML, KEADPTGHSY, SAFPTTINF, DPARYEFLW, ITKKVADLVGF, SAYGEPRKL RVRFFFPSL, SAYGEPRKL, TSCILESLFRAVITK, EYLQLVFGI TSCILESLFRAVITK, PRALAETSYVKVLEY, YLQLVFGIEV, FLLLKYRAREPVTKAE, EYVIKVSARVRF, EYVIKVSARVRF, |
| MAGE-A2 | REPVTKAEML, EGDCAPEEK, EYLQLVFGI,LLKYRAREPVTKAE, EVDPIGHLY, FLWGPRALV, KVAELVHFL, TFPDLESEF, |
| MAGE-A3 | VAELVHFLL, MEVDPIGHLY, EVDPIGHLY, MEVDPIGHLY REPVTKAEML, AELVHFLLL, WQYFFPVIF, EGDCAPEEK |
| MAGE-A3 | KKLLTQHFVQENYLEY, RKVAELVHFLLLKYR, VIFSKASSSLQL KKLLTQHFVQENYLEY, ACYEFLWGPRALVETS RKVAELVHFLLLKYR, VIFSKASSSLQL, FLLLKYRAREPVTKAE, VFGIELMEVDPIGHL, GDNQIMPKAGLLIIV TSYVKVLHHMVKISG, RKVAELVHFLLLKYRA |
| MAGE-A4 | EVDPASNTY, GVYDGREHTV, NYKRCFPVI, SESLKMIF, |
| MAGE-A6 | EVDPIGHVY, REPVTKAEML, EGDCAPEEK, ISGGPRISY LLKYRAREPVTKAE |
| MAGE-A9 | ALSVMGVYV |
| MAGE-A10 | GLYDGMEHLI, DPARYEFLW |
| MAGE-A12 | EGDCAPEEK, REPFTKAEMLGSVIR, VRIGHLYIL, FLWGPRALV, AELVHFLLLKYRAR, |
| MAGE-C1 | SSALLSIFQSSPE, ILFGISLREV, SFSYTLLSL, VSSFFSYTL, KVVEFLAML, |
| MAGE-C2 | LLFGLALIEV, SSTLYLVFSPSSFST, ASSTLYLVF, SESIKKKVL ALKDVEERV, |
| mucin | PDTRPAPGSTAPPAHGVTSA |
| NA88-A | QGQHFLQKV |
| NY-ESO-1 / LAGE-2 | LAAQERRVPR, TVSGNILTIR, APRGPHGGAASGL MPFATPMEA, KEFTVSGNILTI, LAMPFATPM, MLMAQEALAFL, ARGPESRLL, SLLMWITQCFLPVF, SLLMWITQC, ASGPGGGAPR LLEFYLAMPFATPMEAELARRSLAQ, RLLEFYLAMPFA PGVLLKEFTVSGNILTIRLTAADHR, PFATPMEAELARR QGAMLAAQERRVPRAAEVPR, PGVLLKEFTVSGNILTIRLT VLLKEFTVSG, AADHRQLQLSISSCLQQL, KEFTVSGNILTLKEFTVSGNILTIRL, LLEFYLAMPFATPM, AGATGGRGPRGAGA |
| SAGE | LYATVIHDI |
| Sp17 | ILDSSEEDK |
| SSX-2 | KASEKIFYV, EKIQKAFDDIAKYFSK, FGRLQGISPKI WEKMKASEKIFYVYMKRK, KIFYVYMKRKYEAMT |
| SSX-4 | INKTSGPKRGKHAWTHRLRE, KHAWTHRLRERKQLVVYEEI YFSKKEWEKMKSSEKIVYVY, KSSEKIVYVYMKLNYEVMTK MKLNYEVMTKLGFKVTLPPF, LGFKVTLPPFMRSKRAADFH KHAWTHRLRERKQLVVYEEI |
| TAG-1 | SLGWLFLLL, LSRLSNRLL |
| TAG-2 | LSRLSNRLL |
| TRAG-3 | CEFHACWPAFTVLGE |
| TRP2-INT2g | EVISCKLIKR, RQKKIRIQL |
| XAGE-1b/GAGED2a | HLGSRQKKIRIQLRSQ, CATWKVICKSCISQTPG |

| ***Table 4*** | ***Differentiation Antigens*** |
|---|---|
| date of download: 20140309 | http://cancerimmunity.org/peptide/differentiation/ |
| | |

| ***Gene*/*Protein*** | ***Peptide*** |
|---|---|
| | |
| ***CEA*** (gut carcinoma) | YLSGANLNL, IMIGVLVGV, GVLVGVALI, HLFGYSWYK, DTGFYTLHVIKSDLVNEEATGQFRV, YSWRINGIPQQHTQV, QYSWFVNGTF, TYYRPGVNLSLSC, EIIYPNASLLIQN, TYACFVSNL,YACFVSNLATGRNNS, LWWVNNQSLPVSP, AYVCGIQNSVSANRS, EIIYPNASLLIQN, NSIVKSITVSASG |
| ***gp100* / *PmeI17*** (melanoma) | KTWGQYWQV, (A)MLGTHTMEV, ITDQVPFSV, YLEPGPVTA, LLDGTATLRL, VLYRYGSFSV, SLADTNSLAV, RLMKQDFSV, RLPRIFCSC, LIYRRRLMK, ALLAVGATK IALNFPGSQK, ALNFPGSQK, VYFFLPDHL, RTKQLYPEW HTMEVTVYHR, SSPGCQPPA, VPLDCVLYRY, LPHSSSHWL, LPHSSSHWL, SNDGPTLI, GRAMLGTHTMEVTVY, WNRQLYPEWTEAQRLD, TTEWVETTARELPIPEPE, TGRAMLGTHTMEVTVYH, GRAMLGTHTMEVTVY |
| ***mammaglobin-A*** (breast cancer) | PLLENVISK |
| ***Melan-A* / *MART-1*** | AEEAAGIGIL(T), RNGYRALMDKS, (E)AAGIGILTV, |
| (melanoma) | YTTAEEAAGIGILTVILGVLLLIGCWYCRR, EAAGIGILTV, ILTVILGVL, EEAAGIGILTVI, AAGIGILTVILGVL, APPAYEKLpSAEQ, MPREDAHFIYGYPKKGHGHS, RNGYRALMDKSLHVGTQCALTRR, EEAAGIGILTVI, KNCEPVVPNAPPAYEKLSAE |
| ***NY-BR-1*** (breast cancer) | SLSKILDTV |
| ***OA1*** (melanoma) | LYSACFWWL |
| ***PAP*** (prostate cancer) | FLFLLFFWL, TLMSAMTNL, ALDVYNGLL |
| ***PSA*** (prostate carcinoma) | FLTPKKLQCV, VISNDVCAQV |
| ***RAB38* / *NY-MEL-1*** (melanoma) | VLHWDPETV |
| ***TRP-1* / *gp75*** (melanoma) | MSLQRQFLR, SQWRVVCDSLEDYDT, SLPYWNFATG, ISPNSVFSQWRVVCDSLEDYD |
| ***TRP-2*** (melanoma) | SVYDFFVWL, TLDSQVMSL, LLGPGRPYR, ANDPIFVVL, QCTEVRADTRPWSGP, ALPYWNFATG |
| ***tyrosinase*** (melanoma) | KCDICTDEY, SSDYVIPIGTY, MLLAVLYCL, CLLWSFQTSA, YMDGTMSQV, AFLPWHRLF, IYMDGTADFSF, TPRLPSSADVEF, LPSSADVEF, LHHAFVDSIF, QNILLSNAPLGPQFP, SYLQDSDPDSFQD, QCSGNFMGF FLLHHAFVDSIFEQWLQRHRP, SEIWRDIDF |

| ***Table 5*** | ***Antigens Overexpressed in Tumors*** |
|---|---|
| date of download: 20140309 | http://cancerimmunity.org/peptide/overexpressed/ |
| | |

| ***Gene*** | ***Peptide*** |
|---|---|
| | |
| VLRENTSPK | SVASTITGV |
| AIM-2 | RSDSGQQARY |
| ALDH1A1 | LLYKLADLI |
| BCLX (L) | YLNDHLEPWI |
| BING-4 | CQWGRLWQL |
| CALCA | VLLQAGSLHA |
| CD45 | KFLDALISL |
| CPSF | KVHPVIWSL, LMLQNALTTM |
| cyclin D1 | LLGATCMFV, NPPSMVAAGSVVAAV |
| DKK1 | ALGGHPLLGV |
| ENAH (hMena) | TMNGSKSPV |
| EpCAM | RYQLDPKFI |
| EphA3 | DVTFNIICKKCG |
| EZH2 | FMVEDETVL, FINDEIFVEL, KYDCFLHPF, KYVGIEREM, |
| FGF5 | NTYASPRFK |
| glypican-3 | FVGEFFTDV, EYILSLEEL, |
| G250 / MN / CAIX | HLSTAFARV |
| HER-2 / neu | KIFGSLAFL, IISAVVGIL, ALCRWGLLL, ILHNGAYSL, RLLQETELV, VVLGVVFGI, YMIMVKCWMI, HLYQGCQVV, YLVPQQGFFC, PLQPEQLQV, TLEEITGYL, ALIHHNTHL, PLTSIISAV, VLRENTSPK, TYLPTNASL |
| IDO1 | ALLEIASCL |
| IGF2B3 | NLSSAEVVV, RLLVPTQFV |
| IL13Ralpha2 | WLPFGFILI |
| Intestinal carboxyl esterase | SPRWWPTCL |
| alpha-foetoprotein | GVALQTMKQ, FMNKFIYEI, QLAVSVILRV, |
| Kallikrein 4 | FLGYLILGV, SVSESDTIRSISIAS, LLANGRMPTVLQCVN, RMPTVLQCVNVSVVS |
| KIF20A | LLSDDDVVV, AQPDTAPLPV, CIAEQYHTV, |
| Lengsin | FLPEFGISSA |
| M-CSF | LPAVVGLSPGEQEY |
| MCSP | VGQDVSVLFRVTGALQ |
| mdm-2 | VLFYLGQY |
| Meloe | TLNDECWPA, FGRLQGISPKI, CPPWHPSERISSTL |
| MMP-2 | GLPPDVQRV |
| MMP-7 | SLFPNSPKWTSK |
| MUC1 | STAPPVHNV, LLLLTVLTV, PGSTAPPAHGVT |
| MUC5AC | TCQPTCRSL |
| p53 | LLGRNSFEV, SQKTYQGSY, PGTRVRAMAIYKQ, RMPEAAPPV HLIRVEGNLRVE |
| PAX5 | TLPGYPPHV |
| PBF | CTACRWKKACQR |
| PRAME | VLDGLDVLL, SLYSFPEPEA, ALYVDSLFFL, SLLQHLIGL, |
| PSMA | NYARTEDFF |
| RAGE-1 | LKLSGVVRL, PLPPARNGGL, SPSSNRIRNT |
| RGS5 | LAALPHSCL, GLASFKSFLK |
| RhoC | RAGLQVRKNK |
| RNF43 | ALWPWLLMA(T), NSQPVWLCL |
| RU2AS | LPRWPPPQL |
| secernin 1 | KMDAEHPEL |
| SOX10 | AWISKPPGV, SAWISKPPGV |
| STEAP1 | MIAVFLPIV, HQQYFYKIPILVINK |
| survivin | ELTLGEFLKL, TLGEFLKLDRERAKN, |
| Telomerase | ILAKFLHWL, RLVDDFLLV, LTDLQPYMRQFVAHL, RPGLLGASVLGLDDI |
| VEGF | SRFGGAVVR |
| WT1 | TSEKRPFMCAY, CMTWNQMNL, LSHLQMHSRKH, KRYFKLSHLQMHSRKH |

In a further embodiment, a pharmaceutical composition comprises poloxamers in addition such as poloxamer 124, 188, 237, 338, 388 and 407, which e.g., represent a pharmaceutically acceptable carrier. Poloxamers are nonionic triblock copolymers consisting of a central hydrophobic chain of polyoxypropylene (PPO) flanked by two hydrophilic chains of polyoxyethylene (PEO). Poloxamers favour the advantage of a concentration and temperature dependent micelle formation, thermoviscosification and/or gelation. Depending on the PEO and PPO content, selected Poloxamers form injectable sols at temperatures near or below ambient temperature, but exhibit gelation at the body temperature in concentrated solutions that provides the required retardation. In our experiments, a series of Poloxamers of different compositions were extensively investigated regarding their suitability for SD-208 retardation. The formulation technique was systematically optimized. Gel formation, gel stability depending on the environmental conditions, inclusion of drug and injectability were confirmed. Poloxamers are added to stabilize the immune-stimulating cytokine such as IL-2 and GM-CSF, and/or the TGF-beta inhibitor such as ALK-5, and thus, to extend their effect on their target. In another alternative, the combination with one or more poloxamers is for example the basis for a retard formulation, where from the TGF-beta inhibitor and/or the immune-stimulating cytokine and/or the immune-modulating compound are/is released during a certain time period, for example during 12 or 24 h.

### Intratumoral Treatment

In an embodiment, IL-2, GM-CSF and the TGF-beta inhibitor or the pharmaceutical composition, comprising or consisting of these compounds of the present invention, are injected intratumorally. As described (see Background), the TAA phenotype of every individual tumor appears to be the result of genomic instability that generates a unique personal TAA profile. Thus, intratumoral application of drugs for activating the host immune system guides the patients's own tumor to serve as a source of currently expressed, unique TAA profile, i.e., cancer profile. Evaluation of the continuously changing profile of TAA, i.e., of a dynamic TAA profile in a rapidly growing tumor *in vivo* for the purpose of vaccine preparation is technically and logistically impossible. In the present invention, the tumor itself is used to present in real-time the full range of personal TAA in a TGF-beta-free context. Thus, the combined use, e.g., in a pharmacological composition, of a TGF-beta inhibitor in combination with immunostimulating cytokines IL-2 and/or GM-CSF triggers the induction of an antigen-specific immune response against currently available TAA. Intratumoral approach to use a pharmaceutical composition of the present invention generates not only a local anti-tumor immune response but also a systemic reaction by circulating T cells. Employing immune-stimulating cytokine IL-2 and GM-CSF and a TGF-beta inhibitor such as SD-208, e.g., in a pharmaceutical composition, as described, generates not only a local immune response, but indeed also a systemic immune response to the TAA profile in the secondary lesion (metastasis) (Mahwi et al., 2007). Due to a continuous Darwinian selection process in malignant progression of cancer, intratumoral therapy with a pharmaceutical composition, as described in the present invention, is preferably applied repeatedly, whilst always accompanying - in real-time mode - all changes occurring in the TAA profile of the malignant tumor.

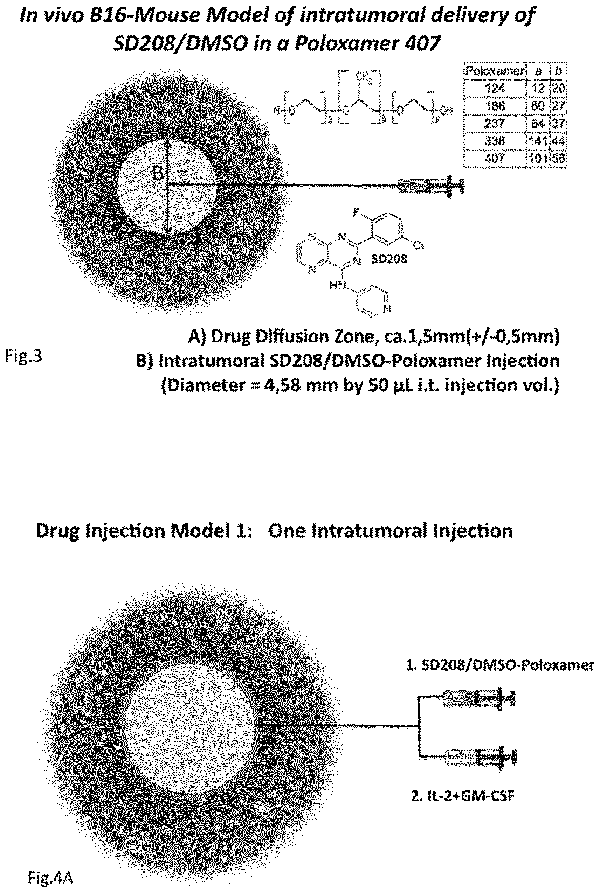

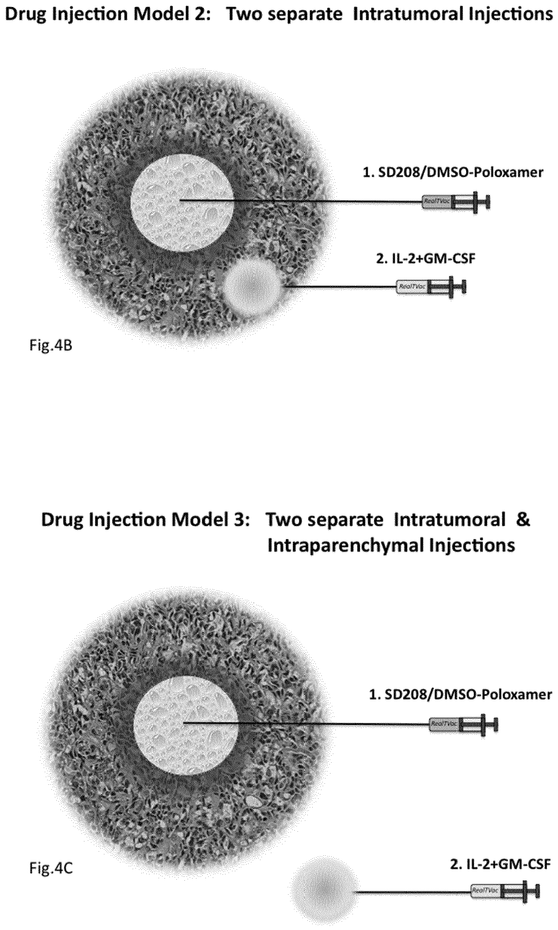

Another important aspect of the current invention is closely related to the significant toxicity associated with systemic application of TGF-beta inhibitors. The vast majority of current drug development programs strongly focus on systemic drug application, mainly driven by the intention to treat disease targets spread throughout the whole body. This also applies to the different systemic approaches, which are currently under investigation to treat cancers by use of anti-TGF-beta therapies, including inhibitors of TGF-beta receptors, TGF-beta targeting antibodies, ligand traps or Inhibitors of TGF-beta synthesis (see e.g., Table1). All these strategies suffer from several limitations with respect to delivery, specificity and/or toxicity. However, their most crucial and common limitation by far is the fact that TGF-beta signaling is involved in many different normal physiological functions, and that normal tissues are as equally compromised as tumors upon systemic effects of anti-TGF-beta therapies. Therefore, the systemic neutralization of this otherwise so important protein is associated with the risk of severe side-effects. In addition, a systemic delivery of TGF-beta inhibitors may hinder the dosing parameters from being maximized because of the potentially harmful side effects. TGF-beta inhibitors may exert their activity in a variety of ways in the treatment of malignant cancer. In most instances, the TGF-beta inhibitor may not target the abnormal cell specifically, but rather exert its activity systemically across all cells. Systemic administration may expose, therefore, both transformed and healthy normal cells to the effects of the TGF-beta inhibitor. Although potentially therapeutically effective against the cancer cells in many ways, systemic administration of TGF-beta inhibitors may cause detrimental side effects to the normal healthy cells. This may result in a smaller amount of the intended dosage of TGF-beta inhibitors reaching and targeting the cancer cells. In addition, a greater amount of the intended dosage of TGF-beta inhibitors may reach the normal healthy cells. Thus, systemic delivery of TGF-beta inhibitors may hinder the dosing parameters from being maximized because of the potentially harmful side effects. The current invention of intratumoral application of a pharmaceutical formulation allows high concentrations of drugs to be achieved within the tumor, whereby this is essential to attain the desired therapeutic effects whilst avoiding systemic toxicity due to overdosing. Preferred concentrations of TGF-beta inhibitors, particularly of ALK-5 inhibitors are for example from about 1 nM to about 10 mM, from about 10 nM to about 10 mM, from about 0.1 µM to about 1 mM, or from about 500 nM to about 5 mM.
To overcome this issue, we invented local (i.e. intratumoral) application of the TGF-beta inhibitor such that efficient concentrations of the anti-TGF-beta drug can be achieved in the growing tumor, while the resulting systemic concentrations are far too low to cause any dose-limiting or clinically relevant toxicity (Fig. 4). The current invention of intratumoral application of the pharmaceutical composition allows achieving high concentrations of drugs within the tumor, which is essential to attain the desired therapeutic effects, while avoiding systemic toxicity due to overdosing. Nevertheless, the real-time vaccination approach can still result in a systemic anti-tumor response by a highly specific, TAA-driven immune response that is amplified and systemically distributed across the body by the circulating T-cells.

The major advantage of the present invention is the combination of simultaneous reduction in TGF-beta function with concomitant activation of the immune cells with IL-2 andGM-CSF. Thus, each individual immune system is able to adapt to the specific tumor situation at the time of the treatment, i.e., in real-time mode and is not limited to pre-selected tumor antigens, as applied in the classic tumor vaccine approach. This is particularly important, as malignant tumors rapidly change their antigenic profile due to a Darwinian selection process. In consequence, the present invention of a combination therapy comprising or consisting of an immunostimulating cytokine, e.g., at least two immune-stimulating cytokines, and a TGF-β inhibitor, when applied intratumorally, always induces maximal therapeutic effect due to the generation of an up-to-date host immune response to the growing tumor. In a one embodiment, the at least two immune-stimulating cytokines IL-2 and GMCSF are combined with an ALK-5 inhibitor such as SD208.

In a further embodiment, the transcription and translation of mRNA, respectively, encoding TGF-beta as well as the interaction of TGF-beta ligands with its receptor strengthen the effect of the immune-stimulating cytokines IL-2 and GM-SCF. Further immune stimulators supporting the effect of IL-2 and/or GM-SCF are selected from the group consisting of neoantigens, other immune-stimulating cytokines such as IFNα, IFNβ, IFNγ, MIPα, MIPβ, TNFα, G-CSF, M-CSF, MIP, and interleukins (IL) such as IL-1α, IL-1β, IL-3, IL-5, IL-6, IL-7, IL-12, IL-15, IL-16, IL-18, IL-21, IL-24, IL-32, IL-33, IL-36, immune-regulating molecules binding to CD40, CD134 (OX40) or CD137, CTLA-4, PD-1, PD-L1 and PD-L2, and/or inhibitors of immune-inhibiting factors such as PGE, IL-10, IL-35, ILT2, ILT3 and/or GITR.

The reduction of the tumor volume and the tumor weight, respectively, by a combination of the present invention is for example in the range of 10 to 90 %, 20 to 80 %, 30 to 60 % or 40 to 50 %. In some embodiments the reduction is 35 or 45 %.

For the purpose of clarity and a concise description, features are described herein as part of the same or separate embodiment. However, it should be understood that the scope of the invention may include embodiments having combinations of all or some of the features described.

### Examples

In the following, examples are provided to specify some aspects of the present invention, but these examples are not considered to limit the scope of the present invention, and the invention is not bound to these examples.

### Example 1

Establishment and culture of primary tumor cell cultures were performed as previously described (Jachimczak et al., 1993). Briefly, tumor specimens were cultivated in Dulbecco's minimum essential medium (DMEM) containing 10% FCS, 1% BME vitamin solution, and 1% MEM nonessential amino acids. HTZ-349 was established as primary cell culture from a patient suffering from glioblastoma multiforme (GBM, Grade IV). A-172 cells were obtained from ATCC (GBM). Peripheral blood mononuclear cells (PBMC) were isolated from venous blood of two healthy donors by standard Ficoll-Hypaque gradient centrifugation (Jachimczak et al., 1993).

SD-208, a small molecule inhibitor of type I kinase of TGF-beta receptor with IC₅₀ = 49 nM, was purchased from Sigma, (Germany). SD-208 inhibits TGF-beta signal transduction in a dose-dependent fashion, as measured by p-SMAD2 western blot analysis (Gaspar et al., 2007).

Interleukin 2 (IL-2, Proleukin S, Aldesleukin), used in this study is a human highly purified protein with a molecular weight of approximately 15,300 daltons, produced by recombinant DNA technology in a genetically engineered *E. coli* strain, purchased from Novartis (Germany). Proleukin S with a specific activity of 1.1mg = 18 million IU was reconstituted in sterile water to a final concentration of 10⁷ IU/ 1ml for further use.

Granulocyte macrophage-colony stimulating factor (GM-CSF, CellGro) is a human recombinant, highly purified protein with a molecular weight of approximately 14.400 daltons produced by recombinant DNA technology using genetically engineered GM-CSF with a specific activity of 50 µg = 4 x 10⁵ IU; this material was purchased from CellGenix GmbH (Germany). CellGro was reconstituted in sterile PBS to a final concentration of 50 µg/ 1ml for further use.

### Example 2

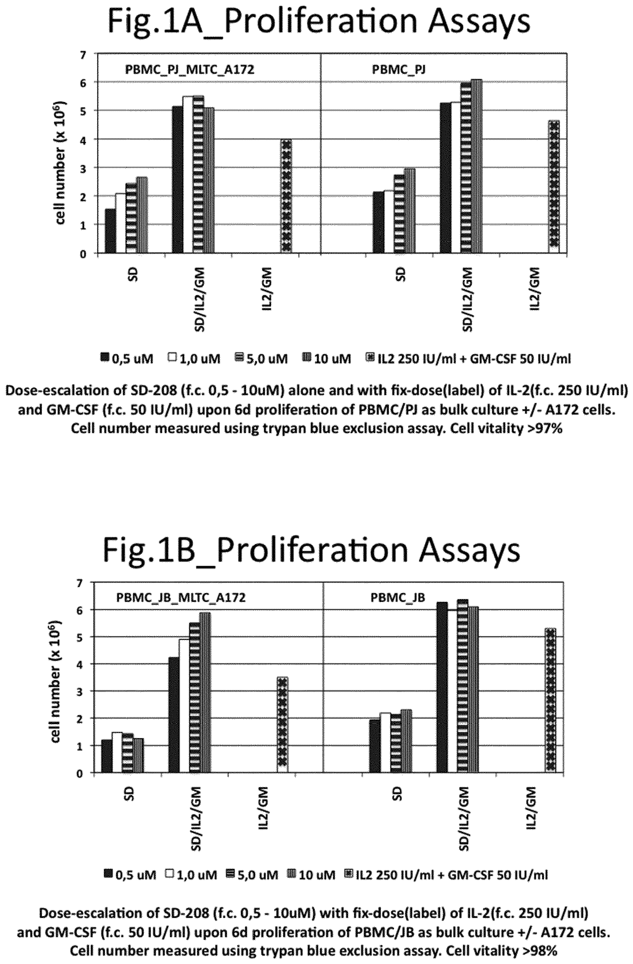

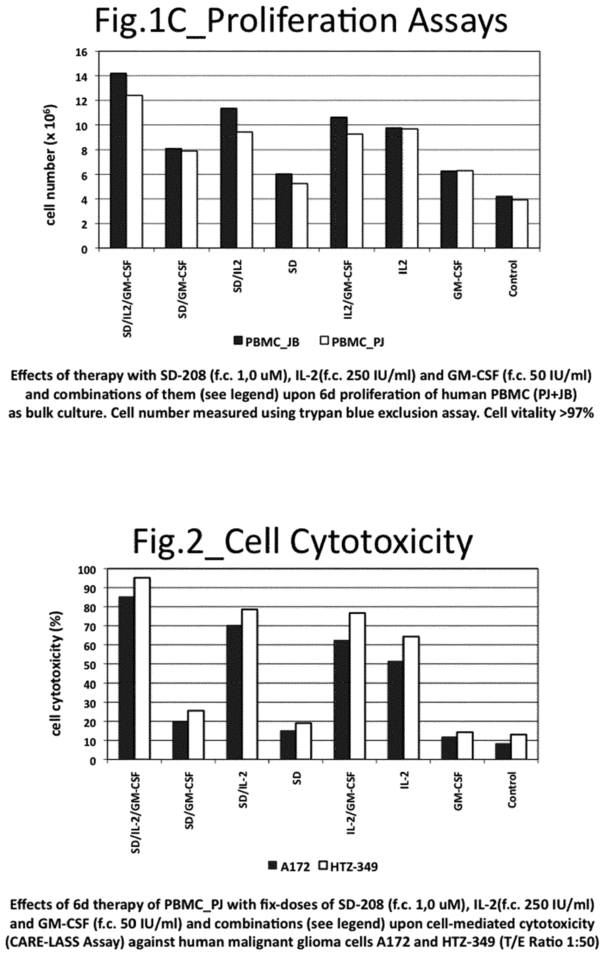

The growth-modulatory effects of various concentrations (SD-208: 0.5-10 µM) were investigated employing a standard proliferation assay (Trypan Blue exclusion). Figures 1 A-C show the relative growth of PBMC treated for 6 days with IL-2, GM-CSF and different concentrations of SD-208. The growth stimulatory effects observed by combined application of IL-2 and GM-CSF were further enhanced by simultaneous treatment with the TGF-beta inhibitor, SD-208.

### Example 3

Cell-mediated tumor cytotoxicity assay (CARE-LASS): In order to evaluate the cytotoxic potential of immune cells treated with TGF-beta inhibitor and immunostimulating cytokines IL-2 and GM-CSF, both effector PBMC and glioma target cells were either preincubated with SD-208 and cytokines or left untreated for 6 days. PBMC were activated with IL-2 (Proleukin S) and GM-CSF. Immune cell cytotoxicity against the target tumor cells was determined in a calcein-release assay (Lichtenfels et al., 1994) by adding pre-activated PBMC to glioma target cells at an effector/target ratio of 1:100, 1:10 and 1:1. After 4 hours, fluorescence of supernatants was analyzed (Fluoroscan, Germany) and calculated as described (Lichtenfels et al., 1994). The allogenous cell cytotoxicity was enhanced by simultaneous tretment with the TGF-beta inhibitor, SD-208 as shown in Fig. 2.

### Example 4

*In vivo* effects of intratumoral application of TGF-beta type I receptor inhibitor, SD 208 with a combination of IL-2 and GM-CSF was investigated in a subcutaneous B16-F10 malignant mouse melanoma model (see Fig. 3). The subcutaneous model is widely used for the evaluation of therapy in many tumor models including B16 melanoma. Upon subcutaneous injection of 10⁵ cells/100µL PBS, B16 form a palpable tumor in 5-10 days and grow to a 1x1x1cm tumor in 14-21 days, or upon subcutaneous injection of 10⁶ cells/150µL PBS, B16 form a palpable tumor in 8-9 days and grow to a 1x1x1cm tumor in approx. 14 days Figures 4A to 4C depict different models of intratumoral injection, wherein Fig. 4A refers to one intratumoral injection, Fig. 4B to two separate intratumoral injections and Fig. 4C to two separate intratumoral/intraparenchymal injections.

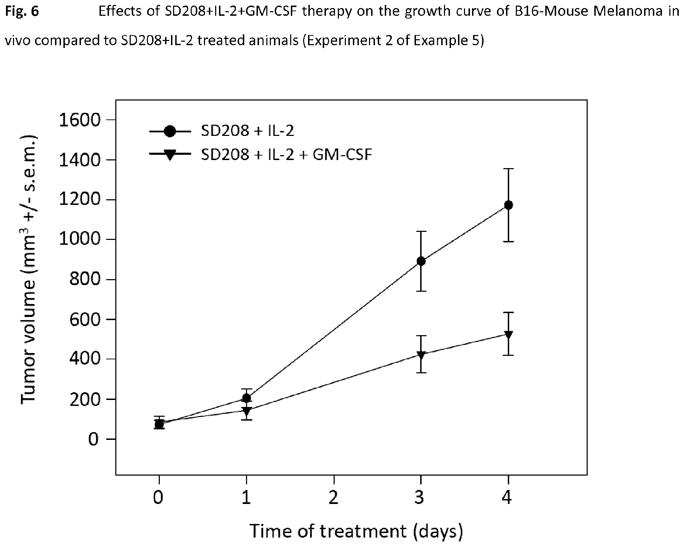

Figures 5A, 5B and 6 present two *in vivo* studies consisted of following experimental groups:
Experiment 1) (Fig. 5A-5D)
   1. Poloxamer 407 + SD-208 (50 µg) + IL-2 + GM-CSF
   2. Control group (poloxamer 407)
Experiment 2) (Fig. 6)
   1. Poloxamer 407 + SD-208 + IL-2
   2. Poloxamer 407 + SD-208 + IL-2 + GM-CSF
   3. Control group (poloxamer 407)
each containig 8-10 animals after randomization.

On day 8 to 9, after a mean tumor volume of approx. 0.5-1.0 ml was reached, 45 tumor-bearing animals were randomized into treatment groups according to tumor sizes. On the same day (day8/9), therapy has been initiated for all groups (Groups 1-2 and 1-3). Each tumor growth was recorded every second day by caliper measurement. On day 13/14 after last treatment the study was terminated, all animals sacrificed and a necropsy performed. At necropsy, animals were weighted and anaesthetized by isoflurane. Blood samples were collected from all animals via retroorbital vein puncture for preparation of serum, which was stored at -20°C for further analysis (e.g., AST(GOT), ALT(GPT), AP, albumin, creatinine). After the blood sampling, animals were killed by cervical dislocation. Primary tumors were collected and wet weights (Fig. 5B) and volumes determined (Fig. 5A and Fig. 6). All primary tumors were snap-frozen in liquid nitrogen and stored appropriately at -80°C. In addition, the liver and kidney(s) were also collected and cryoconserved.

## Claims

1. Combination of a TGF-beta inhibitor selected from the group consisting of an ALK-5 inhibitor, a neutralizing pan-anti-TGF-beta antibody, a neutralizing antibody against TGF-beta-1, or/and -2, or/and -3, a neutralizing anti-TGF-beta receptor type I or/and type II or/and type III antibody or binding fragments thereof, with IL-2 and GM-CSF for use in a method of treating a malignant tumor in a patient, wherein the TGF-beta inhibitor and IL-2 and GM-CSF are injected intratumorally.

2. Combination for use in a method according to claim 1, wherein the combination is injected intratumorally into a tumor metastasis of a malignant tumor.

3. Combination for use in a method according to claim 1 or 2, wherein the combination further comprises an inhibitor of TGF-beta synthesis, selected from the group consisting of antisense oligonucleotides or/and siRNA or/and miRNA or/and antisense RNA, specific against mRNA encoding TGF-beta1, -2, and -3, isotypes.

4. Combination for use in a method according to any one of claims 1-3, wherein the combination further comprises one or more additional immune stimulator(s) selected from the group consisting of IFNα, IFNβ, IFNγ, MIPα, MIPβ, TNFα, G-CSF, M-CSF, MIP, and interleukins (IL) like IL-1α, IL-1β, IL-3, IL-5, IL-6, IL-7, IL-12, IL-15, IL-16, IL-18, IL-21, IL-24, IL-32, IL-33, IL-36.

5. Combination for use in a method according to any one of claims 1-3, wherein the combination comprises one or more additional immune stimulator(s) selected from the group consisting of ligands binding to receptors like CD40, CD134 (OX40) and CD137,TIM-3, immunoglobulin-like transcript (ILT), glucocorticoid-induced tumor necrosis factor family receptor (GITR), and/or inhibitors of prostaglandin E (PGE), interleukin 10 (IL-10), interleukin 35 (IL-35), and/or melanoma inhibiting activity (MIA).

6. Combination for use in a method according to any one of claims 1-3, wherein the combination further comprises one or more additional immune modulators(s) selected from the group consisting of inhibitors of immune-checkpoints like cytotoxic T-lymphocyte antigen 4 (CTLA-4), programed cell death protein 1 (PD-1), or other molecules binding PD-L1 and/or PD-L2.

7. Combination for use in a method according to any one of claims 1-6, wherein the TGF-beta inhibitor, and the immune stimulator are administered at the same time or subsequently.

8. Combination for use in a method according to any one of claims 1-7, wherein the intratumoral concentration of the ALK5 inhibitor is in the range of about 0.1 µM to about 1 mM.

9. Combination for use in a method according to any one of claims 1-8, wherein the intratumoral concentration of the immune stimulator IL-2 is in a range of 1.8x10⁶ to 18x10⁶ (IU) and/or the intratumoral concentration of the immune stimulator GM-CSF is in a range of 0.1-0.5mg.

10. Pharmaceutical composition comprising a combination according to any one of claims 1 to 9 and a pharmaceutically acceptable carrier for use in a method of treating a malignant tumor in a patient, wherein the pharmaceutical composition is injected intratumorally.

11. Pharmaceutical composition for use in a method according to claim 10, wherein the pharmaceutically acceptable carrier is a poloxamer selected from the group consisting of poloxamer 124, 188, 237, 338, 388 and 407.

## Patentansprüche

1. Kombination eines TGF-beta-Inhibitors, ausgewählt aus der Gruppe bestehend aus einem ALK-5-Inhibitor, einem neutralisierenden pan-anti-TGF-beta-Antikörper, einem neutralisierenden Antikörper gegen TGF-beta-1 oder/und -2 oder/und - 3 einen neutralisierenden Anti-TGF-beta-Rezeptor Typ I oder/und Typ II oder/und Typ III Antikörper oder Bindungsfragmente davon, mit IL-2 und GM-CSF zur Verwendung in einem Verfahren zur Behandlung eines malignen Tumors bei einem Patienten, wobei der TGF-beta-Inhibitor und IL-2 und GM-CSF intratumoral injiziert werden.

2. Kombination zur Verwendung in einem Verfahren nach Anspruch 1, wobei die Kombination intratumoral in eine Tumormetastase eines malignen Tumors injiziert wird.

3. Kombination zur Verwendung in einem Verfahren nach Anspruch 1 oder 2, wobei die Kombination weiter einen Inhibitor der TGF-Beta-Synthese umfasst, ausgewählt aus der Gruppe, bestehend aus Antisense-Oligonukleotiden oder/und siRNA oder/und miRNA oder/und Antisense-RNA, spezifisch gegen mRNA, kodierend TGF-beta1,-2 und -3, Isotypen.

4. Kombination zur Verwendung in einem Verfahren nach einem der Ansprüche 1 bis 3, wobei die Kombination weiter einen oder mehrere zusätzliche Immunstimulator(en) umfasst, ausgewählt aus der Gruppe bestehend aus IFNα, IFNβ, IFNγ, MIPα, MIPβ, TNFα, G-CSF, M-CSF, MIP und Interleukine (IL) wie IL-1α, IL-1β, IL-3, IL-5, IL-6, IL-7, IL-12, IL-15, IL-16, IL-18, IL-21, IL-24, IL-32, IL-33, IL-36.

5. Kombination zur Verwendung in einem Verfahren nach einem der Ansprüche 1 bis 3, wobei die Kombination einen oder mehrere zusätzliche Immunstimulator(en) umfasst, ausgewählt aus der Gruppe bestehend aus Liganden, die an Rezeptoren wie CD40, CD134 (OX40) und CD137, TIM-3, Immunoglobulin-ähnliches Transkript (ILT), Glucocorticoid-induzierten Tumor-Nekrose-Faktor-Familienrezeptor (GITR) und/oder Inhibitoren von Prostaglandin E (PGE), Interleukin 10 (IL-10), Interleukin 35 (IL-35) und/oder Melanom-inhibierende Aktivität (MIA) binden.

6. Kombination zur Verwendung in einem Verfahren nach einem der Ansprüche 1 bis 3, wobei die Kombination weiter einen oder mehrere zusätzliche Immunmodulator(en) umfasst, ausgewählt aus der Gruppe , bestehend aus Inhibitoren von Immunkontrollpunkten wie cytotoxisches T-Lymphozytenantigen 4 (CTLA) -4), programmiertes Zelltodprotein 1 (PD-1) oder andere Moleküle, die PD-L1 und/oder PD-L2 binden.

7. Kombination zur Verwendung in einem Verfahren nach einem der Ansprüche 1 bis 6, wobei der TGF-beta-Inhibitor und der Immunstimulator gleichzeitig oder in Folge verabreicht werden.

8. Kombination zur Verwendung in einem Verfahren nach einem der Ansprüche 1 bis 7, wobei die intratumorale Konzentration des ALK5-Inhibitors im Bereich von etwa 0,1 µM bis etwa 1 mM liegt.

9. Kombination zur Verwendung in einem Verfahren nach einem der Ansprüche 1 bis 8, wobei die intratumorale Konzentration des Immunstimulators IL-2 in einem Bereich von 1,8x10⁶ bis 18x10⁶ (IU) und/oder die intratumorale Konzentration des Immunstimulators GM-CSF in einem Bereich von 0,1 bis 0,5 mg liegt.

10. Pharmazeutische Zusammensetzung, umfassend eine Kombination nach einem der Ansprüche 1 bis 9 und einen pharmazeutisch akzeptablen Träger zur Verwendung in einem Verfahren zur Behandlung eines malignen Tumors bei einem Patienten, wobei die pharmazeutische Zusammensetzung intratumoral injiziert wird.

11. Pharmazeutische Zusammensetzung zur Verwendung in einem Verfahren nach Anspruch 10, wobei der pharmazeutisch akzeptable Träger ein Poloxamer ist, ausgewählt aus der Gruppe bestehend aus Poloxamer 124, 188, 237, 338, 388 und 407.

## Revendications

1. Combinaison d'un inhibiteur de TGF-bêta choisi dans le groupe constitué d'un inhibiteur d'ALK-5, un anticorps neutralisant pan-anti-TGF-bêta, un anticorps neutralisant contre TGF-bêta-1 et/ou -2 et/ou -3, un anticorps neutralisant anti-TGF-bêta neutralisant de type I et/ou de type II et/ou de type III, ou des fragments de liaison de ceux-ci, avec l'IL-2 et le GM-CSF, à utiliser dans un procédé de traitement d'une tumeur maligne d'un patient, dans laquelle l'inhibiteur de TGF-bêta et l'IL-2 et le GM-CSF sont injectés par voie intratumorale.

2. Combinaison à utiliser dans un procédé selon la revendication 1, dans laquelle la combinaison est injectée par voie intratumorale dans une métastase tumorale d'une tumeur maligne.

3. Combinaison à utiliser dans un procédé selon la revendication 1 ou 2, dans laquelle la combinaison comprend en outre un inhibiteur de synthèse de TGF-bêta, choisi dans le groupe constitué d'oligonucléotides antisens et/ou d'ARNsi et/ou d'ARNmi et/ou d'ARN antisens, spécifique contre l'ARNm codant pour les isotypes de TGF-bêta-1, -2 et -3.

4. Combinaison à utiliser dans un procédé selon l'une quelconque des revendications 1 à 3, dans laquelle la combinaison comprend en outre un ou plusieurs stimulateur(s) immunitaire(s) supplémentaire(s) choisi(s) dans le groupe constitué d'IFNα, IFNβ, IFNγ, MIPα, MIPβ, TNFα. G-CSF, M-CSF, MIP et interleukines (IL) tels qu'IL-1α, IL-1β, IL-3, IL-5, IL-6, IL-7, IL-12, IL-15, IL-16, IL-18, IL-21, IL-24, IL-32, IL-33, IL-36.

5. Combinaison à utiliser dans un procédé selon l'une quelconque des revendications 1 à 3, dans laquelle la combinaison comprend un ou plusieurs stimulateurs immunitaires supplémentaires choisis dans le groupe constitué de ligands se liant à des récepteurs tels que CD40, CD134 (OX40) et CD137, TIM-3, un transcrit analogue à l'immunoglobuline (ILT), un récepteur de la famille du facteur de nécrose tumorale induit par des glucocorticoïdes (GITR), et/ou des inhibiteurs de la prostaglandine E (PGE), de l'interleukine 10 (IL-10), de l'interleukine 35 (IL-35), et / ou une activité inhibitrice de mélanome (MIA).

6. Combinaison à utiliser dans un procédé selon l'une quelconque des revendications 1 à 3, dans laquelle la combinaison comprend en outre un ou plusieurs modulateurs immunitaires supplémentaires choisis dans le groupe constitué d'inhibiteurs de points de contrôle immunitaires tels que l'antigène 4 des lymphocytes T cytotoxiques (CTLA-4), la protéine de mort cellulaire programmée 1 (PD-1), ou d'autres molécules se liant à PD-L1 et / ou PD-L2.

7. Combinaison à utiliser dans un procédé selon l'une quelconque des revendications 1 à 6, dans laquelle l'inhibiteur de TGF-bêta et le stimulateur immunitaire sont administrés en même temps ou consécutivement.

8. Combinaison à utiliser dans un procédé selon l'une quelconque des revendications 1 à 7, dans laquelle la concentration intratumorale de l'inhibiteur d'ALK5 est dans la gamme d'environ 0,1 µM à environ 1 mM.

9. Combinaison à utiliser dans un procédé selon l'une quelconque des revendications 1 à 8, dans laquelle la concentration intratumorale du stimulateur immunitaire IL-2 se situe dans une plage de 1,8 x 10⁶ à 18 x 10⁶ (IU) et/ou la concentration intratumorale du stimulateur immunitaire GM-CSF se situe dans une plage de 0,1 à 0,5 mg.

10. Composition pharmaceutique comprenant une combinaison selon l'une quelconque des revendications 1 à 9 et un support pharmaceutiquement acceptable à utiliser dans un procédé de traitement d'une tumeur maligne chez un patient, dans laquelle la composition pharmaceutique est injectée par voie intratumorale.

11. Composition pharmaceutique à utiliser dans un procédé selon la revendication 10, dans laquelle le support pharmaceutiquement acceptable est un poloxamère choisi dans le groupe constitué par les poloxamères 124, 188, 237, 338, 388 et 407.
